# EUROPEAN PATENT SPECIFICATION

(11) **EP 2 959 943 B1**
(45) Date of publication and mention of the grant of the patent: **19.10.2016**
(21) Application number: 15173548.7
(22) Date of filing: 24.06.2015
(51) Int. Cl.: A61N 5/06

(54) **OPTICAL HAIR-GROWING DEVICE**
OPTISCHE HAARWACHSTUMSVORRICHTUNG
DISPOSITIF DE CROISSANCE PILEUSE OPTIQUE

(30) Priority: 27.06.2014 JP 2014133160
(43) Date of publication of application: 30.12.2015
(73) Proprietor: Panasonic Intellectual Property Management Co., Ltd., Osaka-shi, Osaka 540-6207 (JP)
(72) Inventor: TAKEUCHI, Toshihiro, Osaka-shi, Osaka 540-6207 (JP); SHIBA, Takeshi, Osaka-shi, Osaka 540-6207 (JP)
(74) Representative: Grünecker Patent- und Rechtsanwälte PartG mbB

(56) References cited:
- KR-B1- 101 288 406
- US-A1- 2010 076 529
- US-A1- 2011 015 707
- US-A1- 2013 041 432
- US-A1- 2013 066 404

## Description

### BACKGROUND OF THE INVENTION

### 1. Field of the Invention

The present disclosure relates to an optical hair-growing device that promotes hair growing by using light.

### 2. Description of the Related Art

There has been known an optical hair-growing device that is attached on the head of a user and outputs light toward the head to promote hair growing. This type of optical hair-growing device includes a headset having, for example, a headphone-like shape, and a controller that operates the headset. The headset includes a light emitting unit that outputs light, a pair of arms attached to the light emitting unit, and a pair of attachments each attached to an end of each of the arms.

The user attaches the headset on his or her head, by which the pair of attachments comes into contact with side head portions so as to sandwich the head, the arms are arranged along the head, and the light emitting unit is opposed to an object site of the head. When the controller is operated, the light emitting unit starts an operation to supply the light output from the light emitting unit to the object site of the head. WO 2004/026400 has disclosed one example of the above-described optical hair-growing device.

US 2013/0041432 A1 discloses a eyebrow hair growth promotion device that has brow plates with light generating sources. Moreover, US 2013/0041432 A1 describes two arm members which are attached to the brow plates and a pair of ear pieces which are connected with two arm members. Furthermore, US 2013/0041432 A1 discloses that the ear pieces are configured to wrap around the user's ears. In addition, nose pads which are described in US 2013/0041432 A1 are attached to a noise bridge that is connected with the brow plates.

### SUMMARY OF THE INVENTION

The light emitting unit has a larger weight because it includes a light source and the like. Thus, even if a user properly attaches the headset on the head before using the optical hair-growing device, there is a possibility that the light emitting unit moves with respect to the head due to its own weight of the light emitting unit during the use of the optical hair-growing device.

If the light emitting unit moves with respect to the head, a part or all of the light output from the light emitting unit is disabled to reach the object site of the head, so that an effect of promoting the hair growing may be deteriorated, or may substantially not be obtained.

An object of the present disclosure is to provide an optical hair-growing device in which a position of a light emitting unit with respect to a head is hard to be displaced.

An optical hair-growing device according to one exemplary embodiment of the present disclosure includes a light emitting unit that outputs light, at least one arm attached to the light-emitting unit, and a pair of first attachments attached to the arm to produce reaction force, and at least one second attachment attached to the light emitting unit or the arm to produce reaction force, and a pair of joints that connects the arm and the first attachments; wherein the optical hair-growing device (10) further comprises at least one of a structure in which each of the joints can rotate with respect to the arm about a first rotation axis extending in the same direction or in substantially the same direction as a longitudinal direction of the arm, and a structure in which each of the first attachments can rotate with respect to each of the joints about a second rotation axis extending in the same direction or in substantially the same direction as a lateral direction of the arm. According to the present exemplary embodiment, the position of the light emitting unit with respect to the head is hard to be displaced.

### BRIEF DESCRIPTION OF DRAWINGS

FIG. 1 a front view of an optical hair-growing device of a first exemplary embodiment;
FIG. 2 is a front view of an optical hair-growing device of a second exemplary embodiment;
FIG. 3 is a front view of an optical hair-growing device of a third exemplary embodiment;
FIG. 4 is a front view of an optical hair-growing device of a fourth exemplary embodiment;
FIG. 5 is a front view of an optical hair-growing device of a fifth exemplary embodiment;
FIG. 6 is a front view of an optical hair-growing device of a sixth exemplary embodiment;
FIG. 7 is a front view of an optical hair-growing device of a seventh exemplary embodiment;
FIG. 8 is a side view of the optical hair-growing device in FIG. 7;
FIG. 9 is a plan view of the optical hair-growing device in FIG. 7;
FIG. 10 is a bottom view of the optical hair-growing device in FIG. 7;
FIG. 11 is an exploded perspective view of a light emitting unit and the like in FIG. 7;
FIG. 12 is an exploded perspective view of a left arm and the like in FIG. 7;
FIG. 13 is an exploded perspective view of a right arm and the like in FIG. 7;
FIG. 14 is an exploded perspective view of a controller in FIG. 7;
FIG. 15 is a cross-sectional view along X15 -X15 in FIG. 9;
FIG. 16 is an enlarged view of a part of FIG. 15;
FIG. 17 is a cross-sectional view along X17 -X17 in FIG. 9;
FIG. 18 is a cross-sectional view along X18 -X18 in FIG. 7;
FIG. 19 is a cross-sectional view showing a flow of air in the light emitting unit in FIG. 16;
FIG. 20A is a front view of the arm and the like in FIG. 7;
FIG. 20B is a front view showing a state where a slider in FIG. 20A contracts;
FIG. 21A is a front view showing a state where a headset in FIG. 7 is attached to a head; and
FIG. 21B is a side view of FIG. 21A.

### DETAILED DESCRIPTION OF THE PREFERRED EMBODIMENTS

### (Exemplary Embodiments that Optical Hair-Growing Device Can Take)

[1] An optical hair-growing device according to a first exemplary embodiment of the present disclosure includes a light emitting unit that outputs light, at least one arm attached to the light-emitting unit, a pair of first attachments attached to the arm to produce reaction force, and at least one second attachment attached to the light emitting unit or the arm to produce reaction force.
   When the present optical hair-growing device is attached to a head, the following state may be formed. According to the state, the pair of first attachments sandwiches the head, and further, the second attachment comes into contact with the head, and the light emitting unit is opposed to the head. In this case, the pair of first attachments and the second attachment each give the reaction force to the head. Thus, as compared with a case where the second attachment does not exist, the reaction force that the optical hair-growing device gives to the head is larger, and a position of the light emitting unit with respect to the head is harder to be displaced.
   One of characteristic technical ideas included in the present disclosure is that in view of the inclusion of the light emitting unit, which is a heavy load, in the optical hair-growing device, the reaction force capable of firmly maintaining the position of the heavy load is given to the head. The second attachment included in the above-described optical hair-growing device corresponds to one example of a technical item on which the technical idea is reflected.
[2] In the optical hair-growing device according to a second exemplary embodiment of the present disclosure, the second attachment is attached to the light emitting unit or the arm so as not to disturb travel of the light output from the light emitting unit.
   According to the present embodiment, the second attachment comes into contact with the head so as not to disturb the travel of the light output from the light emitting unit, and maintain the position of the light emitting unit with respect to the head. Therefore, the light is efficiently supplied to the head, as compared with a case where the light emitting unit does not include the constitution according to the present exemplary embodiment.
[3] In the optical hair-growing device according to a third exemplary embodiment of the present disclosure, a pair of arms is included, the pair of arms includes a left arm and a right arm, one of the first attachments is attached to the left arm, the other first attachment is attached to the right arm, and a left hinge connecting the left arm and the light emitting unit, and a right hinge connecting the right arm and the light emitting unit are further included.
   The left hinge rotates the left arm with respect to the light emitting unit so as to change a distance between the one first attachment and the other first attachment, and the right hinge rotates the right arm with respect to the light emitting unit so as to change the distance between the one first attachment and the other first attachment.
   According to the present exemplary embodiment, when the optical hair-growing device is attached on the head, the pair of arms rotates with respect to the light emitting unit, by which the distance between the pair of first attachments changes in accordance with a shape of the head. Thus, the pair of first attachments can firmly maintain the head even when being attached to each of the heads having various shapes. This makes the position of the light emitting unit with respect to the head still harder to be displaced.
[4] In the optical hair-growing device according to a fourth exemplary embodiment of the present disclosure, the second attachment generates the reaction force of a predetermined value or more when the second attachment is elastically deformed.
   When the optical hair-growing device is attached on the head, in some shapes of the head, the pair of first attachments comes into firm contact with the head, while the second attachment may be hard to come into firm contact with the head. Thus, according to a different exemplary embodiment that the optical hair-growing device can take, that is, according to a form in which the constitution shown in the present exemplary embodiment is not included in the second attachment, there is a possibility that by the second attachment, the effect of maintaining the position of the light emitting unit with respect to the head cannot be obtained.
   In order to solve or alleviate the above-described problem, the optical hair-growing device according to the present exemplary embodiment includes the second attachment constituted as described above. Thus, even if the head has a shape hard to come into firm contact with the second attachment, enough contact of the second attachment with the head to slightly elastically deform the second attachment allows the second attachment to generate the reaction force of the predetermined value or more.
   When the second attachment generates the reaction force of the predetermined value or more, the second attachment substantially contributes to suppression of the displacement of the light emitting unit with respect to the head. That is, when the second attachment comes into contact with the head, the role of the second attachment is substantially played. Thus, the optical hair-growing device enables the position of the light emitting unit with respect to the head to be firmly maintained even when attached on each of the heads having various shapes.
[5] In the optical hair-growing device according to a fifth exemplary embodiment of the present disclosure, the second attachment is attached to the light emitting unit, and force with which the first attachments maintain their own positions is set larger than force that is to move the first attachments, based on the reaction force that the second attachment produces.
   According to the present exemplary embodiment, the second attachment gives the reaction force to the head, by which force in a direction where the second attachment is separated from the head acts on the second attachment. Thus, if the movement of the second attachment based on the force cannot be suppressed, the arm and the light-emitting unit also move with respect to the head, accompanying the movement of the second attachment. In this case, a distance between the head and the light emitting unit is increased, so that the light output from the light emitting unit becomes hard to reach the head.
   In order to solve or alleviate the above-described problem, the optical hair-growing device according to the present exemplary embodiment includes the pair of first attachments constituted as described above. According to the present optical hair-growing device, even if the force separating the second attachment from the head acts on the second attachment, the first attachments substantially do not move with respect to the head.
   Thus, the second attachment substantially does not move with respect to the head, either, and the distance between the head and the light emitting unit is prevented from being increased. Therefore, as compared with a case where the optical hair-growing device does not include the above constitution [5], the light can be efficiently supplied from the light emitting unit to the head.
[6] In the optical hair-growing device according to a sixth exemplary embodiment of the present disclosure, the arm further includes a telescopic structure that changes a length of the arm itself, and a lock mechanism that maintains the length of the arm, and force generated by the lock mechanism to maintain the length of arm is set larger than force that extends the arm, based on the reaction force that the second attachment produces.
   According to the optical hair-growing device including the second attachment, the second attachment gives the reaction force to the head, by which the force in the direction where the second attachment is separated from the head acts on the second attachment. Thus, if the extension of the arm based on the force cannot be suppressed, the light emitting unit moves with respect to the head, accompanying the extension of the arm. In this case, the distance between the head and the light emitting unit is increased, and the light output from the light emitting unit becomes hard to reach the head.
   In order to solve or alleviate the above-described problem, the optical hair-growing device according to the present exemplary embodiment includes the lock mechanism constituted as described above. According to the present hair-growing device, even if the force separating the second attachment from the head acts on the second attachment, the arm substantially does not extend. Thus, the light emitting unit substantially does not move with respect to the head, and the distance between the head and the light emitting unit is prevented from being increased, so that the light is efficiently supplied from the light emitting unit to the head.
[7] The optical hair-growing device according to a seventh exemplary embodiment of the present disclosure further includes a pair of joints that connects the arm and the first attachments, and at least one of a structure in which each of the joints can rotate with respect to the arm about a first rotation axis extending in the same direction or in substantially the same direction as a longitudinal direction of the arm, and a structure in which each of the first attachments can rotate with respect to the joint about a second rotation axis extending in the same direction or in substantially the same direction as a lateral direction of the arm.

According to the present exemplary embodiment, in the case where the structure is included in which each of the joints rotates with respect to the arm about the first rotation axis, when the optical hair-growing device is attached on the head, each of the joints and each of the first attachments rotate with respect to the arm about the first rotation axis in accordance with a shape in a front-back direction of a side head portion. Thus, even if the optical hair-growing device is attached on each of the heads having various shapes, the first attachments easily come into firm contact with the side head portions.

According to the present exemplary embodiment, in the case where the structure is included in which each of the first attachments rotates with respect to each of the joints about the second rotation axis, when the optical hair-growing device is attached on the head, each of the first attachments rotates with respect to each of the joints about the second rotation axis in accordance with a shape in a vertical direction of the side head portion. Thus, even if the optical hair-growing device is attached on each of the heads having various shapes, the first attachments easily come into firm contact with the side head portions.

### (First Exemplary Embodiment)

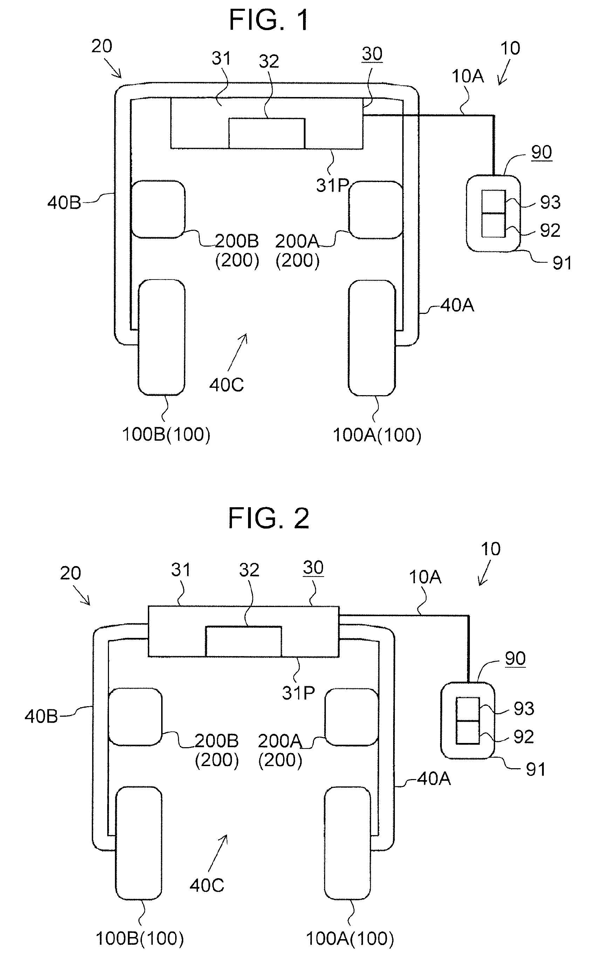

FIG. 1 shows optical hair-growing device 10 of a first exemplary embodiment. Optical hair-growing device 10 includes headset 20 that supplies light to an object site, controller 90 to operate headset 20, and cord 10A that connects headset 20 and controller 90.

Headset 20 includes light emitting unit 30 that outputs light, a pair of arm 40 to which light emitting unit 30 is attached, a pair of first attachments 100 attached to arm 40, and a pair of second attachments 200 attached to arm 40.

Headset 20 further includes a first reaction-force applying structure that allows first attachments 100 to produce reaction force. One example of the first reaction-force applying structure includes an elastic element (illustration is omitted) arranged in an interior of arm 40.

The first reaction-force applying structure gives the reaction force to a head through first attachments 100 by deformation of the elastic element caused by attaching headset 20 on the head. This reaction force maintains positions of first attachments 100 with respect to the head. One example of the elastic element constituting the first reaction-force applying structure is a spring.

Headset 20 further includes a second reaction-force applying structure that allows second attachments 200 to produce reaction force. One example of the second reaction-force applying structure includes an elastic element (illustration is omitted) arranged in an interior of each of second attachments 200.

The second reaction-force applying structure gives the reaction force to a head by the deformation of the elastic element caused by attaching headset 20 on the head, and this reaction force maintains positions of second attachments 200 with respect to the head. One example of the elastic element constituting the second reaction-force applying structure is a spring.

Arm 40 has a U shape or an analogous shape to the U shape. The shape of arm 40 forms arrangement space 40C inside arm 40. The head of a user is arranged in arrangement space 40C.

Light emitting unit 30 includes unit housing 31 that forms an outer shape of light emitting unit 30, and light source 32 arranged in an interior of unit housing 31. Unit housing 31 includes opposed surface 31P. Opposed surface 31P faces arrangement space 40C. Light source 32 faces arrangement space 40C as with opposed surface 31P. Light output from light source 32 is light that can contribute to promotion of hair growing of the head, and one example of the light is near-infrared ray.

The pair of first attachments 100 is composed of left first attachment 100A attached to a left portion with respect to a center of arm 40, and right first attachment 100B attached to a right portion with respect to the center of arm 40. The center of arm 40 is substantially coincident with a center in a right-left direction, which is a width direction of headset 20.

The pair of second attachments 200 is composed of left second attachment 200A attached to a left portion with respect to the center of arm 40, and right second attachment 200B attached to a right portion with respect to the center of arm 40.

Left second attachment 200A is attached to arm 40 between left first attachment 100A and light emitting unit 30. Right second attachment 200B is attached to arm 40 between right first attachment 100B and light emitting unit 30. Second attachments 200 are attached to arm 40 so as not to disturb travel of the light output from light source 32 of light emitting unit 30.

Controller 90 includes case 91 that forms an outer shape of controller 90, power supply 92 that supplies electric power to light emitting unit 30, and circuit board 93 that controls operation of light emitting unit 30. Power supply 92 and circuit board 93 are arranged in an interior of case 91, and are connected to light emitting unit 30 by cord 10A. Light source 32 outputs the light by the electric power supplied from controller 90.

Optical hair-growing device 10 is used, for example, as follows.

The head is arranged in arrangement space 40C of headset 20, and headset 20 is attached on the head, by which, for example, a following state is formed. The pair of first attachments 100 comes into contact with side head portions so as to cover respective ears, and deformation of arm 40 caused by the contact with the side head portions allows the first reaction-force applying structure to supply the reaction force to the head through first attachments 100. The pair of first attachments 100 sandwiches the head cooperatively.

The pair of second attachments 200 comes into contact with the respective side head portions, and elastic deformation of second attachments 200 caused by the contact with the side head portions allows the second reaction-force applying structure to give the reaction force to the head through second attachments 200. The pair of second attachments 200 sandwiches the head cooperatively.

Light emitting unit 30 is arranged around an object site of the head. The object site of the head is, for example, a head top. Opposed surface 31P and light source 32 face the object site. The user can arbitrarily change the object site of the head by changing a position of light emitting unit 30 with respect to the head.

When the user starts operation of light emitting unit 30 by operating controller 90 after attaching headset 20 on his or her head, the light is output from light source 32. The light output from light source 32 is supplied to the object site of the head. This may bring about an effect of promoting hair growing.

According to optical hair-growing device 10 of the first exemplary embodiment, the following effects can be obtained.
(1) Optical hair-growing device 10 includes at least one second attachment 200. Thus, as compared with a case where second attachment 200 does not exist, the reaction force applied to the head by headset 20 is larger, which makes unlikely displacement of the position of light emitting unit 30 with respect to the head.
(2) According to the constitution of the above-described (1), even when the user attaches headset 20 for a long time, a position of headset 20 with respect to the head is hard to be displaced. Thus, the light is stably supplied to the object site of the head, so that the effect of promoting the hair growing may be further increased.
(3) Second attachments 200 are attached to arm 40 so as not to disturb the travel of the light output from light source 32 of light emitting unit 30.

According to this constitution, when headset 20 is attached on the head, second attachments 200 come into contact with the head so as not to disturb the travel of the light output from light source 32, and maintain the position of light emitting unit 30 with respect to the head. This allows the light to be efficiently supplied from light emitting unit 30 to the head, as compared with a case where second attachments 200 are not arranged as described above.

### (Second Exemplary Embodiment)

FIG. 2 shows optical hair-growing device 10 of a second exemplary embodiment. Optical hair-growing device 10 of the second exemplary embodiment includes a constitution in which a part of optical hair-growing device 10 of the first exemplary embodiment is changed as follows. Elements given reference numerals common to the first exemplary embodiment have similar or analogous functions to the corresponding elements of the first exemplary embodiment.

Headset 20 includes left arm 40A and right arm 40B, which makes up a pair of arms 40. Left arm 40A is connected to a left end of light emitting unit 30. Right arm 40B is connected to a right end of light emitting unit 30. Left first attachment 100A and left second attachment 200A are attached to left arm 40A. Right first attachment 100B and right second attachment 200B are attached to right arm 40B.

### (Third Exemplary Embodiment)

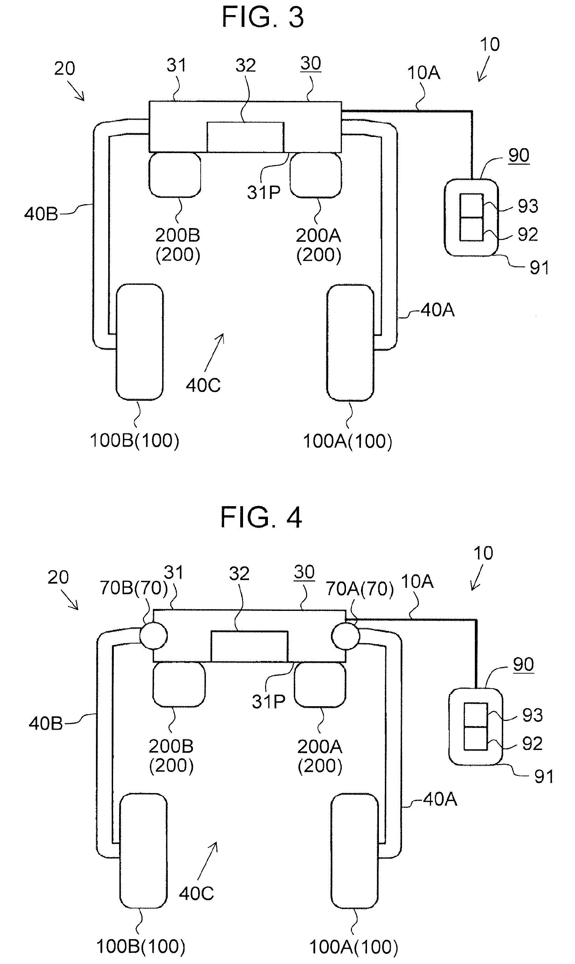

FIG. 3 shows optical hair-growing device 10 of a third exemplary embodiment. A constitution of optical hair-growing device 10 of the third exemplary embodiment corresponds to a constitution in which a part of optical hair-growing device 10 of the second exemplary embodiment is changed as follows. Elements given reference numerals common to the second exemplary embodiment have similar or analogous functions to the corresponding elements of the second exemplary embodiment.

The pair of second attachments 200 is attached to opposed surface 31P of unit housing 31. Headset 20 is attached on a head, which brings the pair of second attachments 200 into contact with a head top portion. A second reaction-force applying structure gives reaction force to the head through second attachments 200.

At this time, force (hereinafter, referred to as "displacement promoting force") that is to move each of second attachments 200 with respect to the head, based on the reaction force given to the head, acts on second attachments 200 and first attachments 100.

Force that causes each of first attachments 100 to maintain its own position with respect to the head (hereinafter, referred to as "maintaining force of first attachment 100") is set larger than displacement promoting force acting on first attachment 100. This substantially prevents first attachments 100 from moving with respect to the head even when the displacement promoting force acts on second attachments 200.

The maintaining force of first attachments 100 is mainly decided by friction force occurring between first attachments 100 and the head. This friction force is mainly decided by the reaction force that the first reaction-force applying structure gives to the head through first attachments 100. According to one example, the reaction force that the first reaction-force applying structure gives to the head through first attachments 100 is set larger than the reaction force that second attachments 200 gives to the head, by which the above-described maintaining force of first attachments 100 is obtained.

According to optical hair-growing device 10 of the third exemplary embodiment, the following effects can be obtained in addition to the effects of (1) to (3) obtained by the optical hair-growing device 10 of the second exemplary embodiment.
(4) Optical hair-growing device 10 can take various forms different from the form exemplified in the exemplary embodiment. According to one example of the different forms, the maintaining force of first attachments 100 is smaller than the displacement promoting force.

Therefore, when headset 20 is attached on the head, movement of second attachments 200 based on the displacement promoting force cannot be suppressed, so that arms 40 and light emitting unit 30 may move with respect to the head, accompanying the movement of second attachments 200. In this case, a distance between the head and light emitting unit 30 is increased, and the light output from light source 32 becomes hard to reach the head.

According to optical hair-growing device 10 of the third exemplary embodiment, in order to solve or alleviate the above-described problem, the maintaining force of first attachments 100 is set as described above.

According to this constitution, even if the displacement promoting force acts on second attachments 200, first attachments 100 substantially does not move with respect to the head by the maintaining force of first attachments 100. Thus, second attachments 200 substantially do not move with respect to the head, either, and light emitting unit 30 is prevented from moving with respect to the head. Therefore, as compared with the above-described different forms, the light output from light source 32 is efficiently supplied to the object site of the head.

### (Fourth Exemplary Embodiment)

FIG. 4 shows optical hair-growing device 10 of a fourth exemplary embodiment. Optical hair-growing device 10 of the fourth exemplary embodiment further includes the following constitution, which is not described in the third exemplary embodiment. Elements given reference numerals common to the third exemplary embodiment have similar or analogous functions to the corresponding elements of the third exemplary embodiment.

Headset 20 further includes left hinge 70A and right hinge 70B, which make up a pair of hinges 70. Hinges 70 each have a rotation axis extending in a substantially same direction as a front-back direction of headset 20.

Left hinge 70A connects between left arm 40A and a left end of light emitting unit 30 so that left arm 40A can rotate with respect to light emitting unit 30 about the rotation axis. Left arm 40A rotates with respect to light emitting unit 30, by which a position of left first attachment 100A with respect to right first attachment 100B is changed so that a distance between the pair of first attachments 100 is changed.

Right hinge 70B connects between right arm 40B and a right end of light emitting unit 30 so that right arm 40B can rotate with respect to light emitting unit 30 about the rotation axis. Right arm 40B rotates with respect to light emitting unit 30, by which a position of right first attachment 100B with respect to left first attachment 100A is changed so that the distance between the pair of first attachments 100 is changed.

According to optical hair-growing device 10 of the fourth exemplary embodiment, the following effects can be obtained in addition to the effects of (1) to (4) obtained by optical hair-growing device 10 of the third exemplary embodiment.
(5) Optical hair-growing 10 includes a pair of hinges 70. According to this constitution, the distance between the pair of first attachments 100 is changed when headset 20 is attached on the head.

This enables the pair of first attachments 100 to firmly sandwich the head even when first attachments 100 are attached on each of the heads having various shapes. As a result, a position of light emitting unit 30 with respect to the head becomes hard to be displaced.

### (Fifth Exemplary Embodiment)

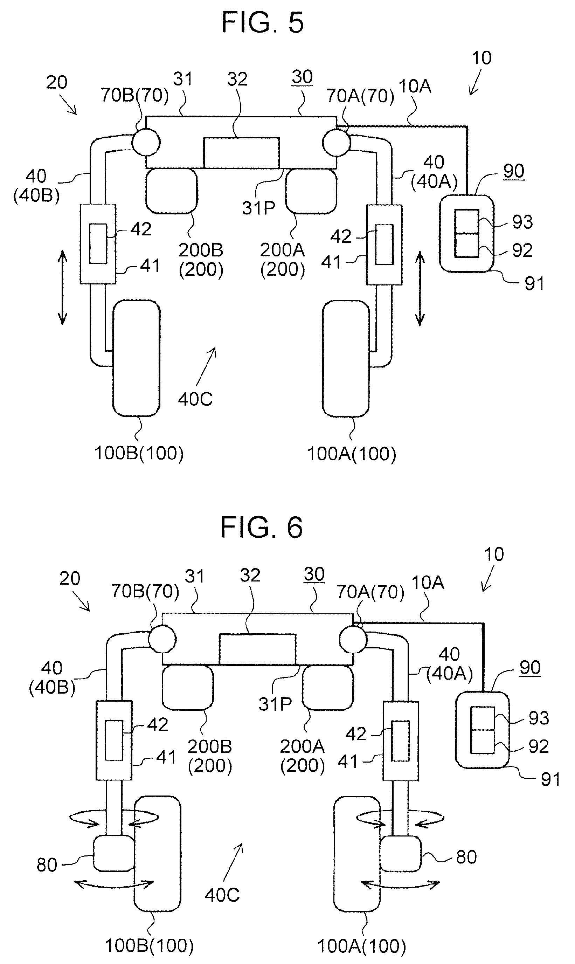

FIG. 5 shows optical hair-growing device 10 of a fifth exemplary embodiment. Optical hair-growing device 10 of the fifth exemplary embodiment further includes the following constitution, which is not described in the fourth exemplary embodiment. Elements given reference numerals common to the fourth exemplary embodiment have similar or analogous functions to the corresponding elements of the fourth exemplary embodiment.

Headset 20 further includes telescopic structures 41 that change a length of arms 40, and lock mechanisms 42 that maintain the length of arms 40. When telescopic structures 41 extend arms 40, first attachments 100 move downward with respect to light emitting unit 30.

When headset 20 is attached on the head, the displacement promoting force acts on second attachments 200, and force (hereinafter, referred to as "extension promoting force") that extends arms 40, based on this displacement promoting force may act on arms 40. Force that causes lock mechanisms 42 to maintain the length of arms 40 (hereinafter, referred to as "maintaining force of lock mechanisms 42") is set larger than the extension promoting force acting on arms 40, based on the displacement promoting force.

According to optical hair-growing device 10 of the fifth exemplary embodiment, the following effects can be obtained in addition to the effects of (1) to (5) obtained by optical hair-growing device 10 of the fourth exemplary embodiment.
(6) Optical hair-growing device 10 can take various forms different from the form exemplified in the exemplary embodiment. According to one example of the different forms, the maintaining force of lock mechanisms 42 is smaller than the extension promoting force. Therefore, arms 40 may be extended by the extension promoting force acting on arms 40, based on the displacement promoting force. In this case, the distance between the head and light emitting unit 30 is increased, and the light output from light source 32 becomes hard to reach the head.

According to optical hair-growing device 10 of the fifth exemplary embodiment, in order to solve or alleviate the above-described problem, the maintaining force of lock mechanisms 42 is set as described above. According to this constitution, even if the extension promoting force acts on arms 40, the maintaining force of lock mechanisms 42 substantially prevents arms 40 from extending, so that the position of light emitting unit 30 with respect to the head can be maintained. Thus, as compared with the above-described different forms, the light output from light source 32 is efficiently supplied to the object site of the head.

### (Sixth Exemplary Embodiment)

FIG. 6 shows optical hair-growing device 10 of a sixth exemplary embodiment. Optical hair-growing device 10 of the sixth exemplary embodiment further includes the following constitution, which is not described in the fifth exemplary embodiment. Elements given reference numerals common to the fifth exemplary embodiment have similar or analogous functions to the corresponding elements of the fifth exemplary embodiment.

Headset 20 further includes a pair of joints 80 each connecting between arm 40 and first attachment 100. Joints 80 each have a first rotation axis extending in the same direction or substantially the same direction as a longitudinal direction of arm 40 and a second rotation axis extending in the same direction or substantially the same direction as a width direction of first attachment 100.

Joints 80 are each attached to arm 40 so as to be rotatable with respect to arm 40 about the first rotation axis. First attachments 100 are each attached to joint 80 so as to be rotatable together with joint 80 with respect to arm 40 about the first rotation axis, and so as to be rotatable with respect to joint 80 about the second rotation axis.

According to optical hair-growing device 10 of the sixth exemplary embodiment, the following effects can be obtained in addition to the effects of (1) to (6) obtained by optical hair-growing device 10 of the fifth exemplary embodiment.
(7) Optical hair-growing 10 includes the structure in which joints 80 each rotate with respect to arm 40 about the first rotation axis. According to this constitution, when headset 20 is attached on the head, each of joints 80 and each of first attachments 100 rotate about the first rotation axis in accordance with a shape in a front-back direction of a side head portion. Therefore, even when headset 20 is attached on each of the heads having various shapes, first attachments 100 easily come into firm contact with the side head portions.
(8) Optical hair-growing device 10 includes the structure in which first attachments 100 each rotate with respect to joint 80 about the second rotation axis. According to this constitution, when headset 20 is attached on the head, first attachments 100 each rotate about the second rotation axis in accordance with a shape in a vertical direction of the side head portion. Therefore, even when headset 20 is attached on each of the heads having various shapes, first attachments 100 easily come into firm contact with the side head portions.

### (Seventh Exemplary Embodiment)

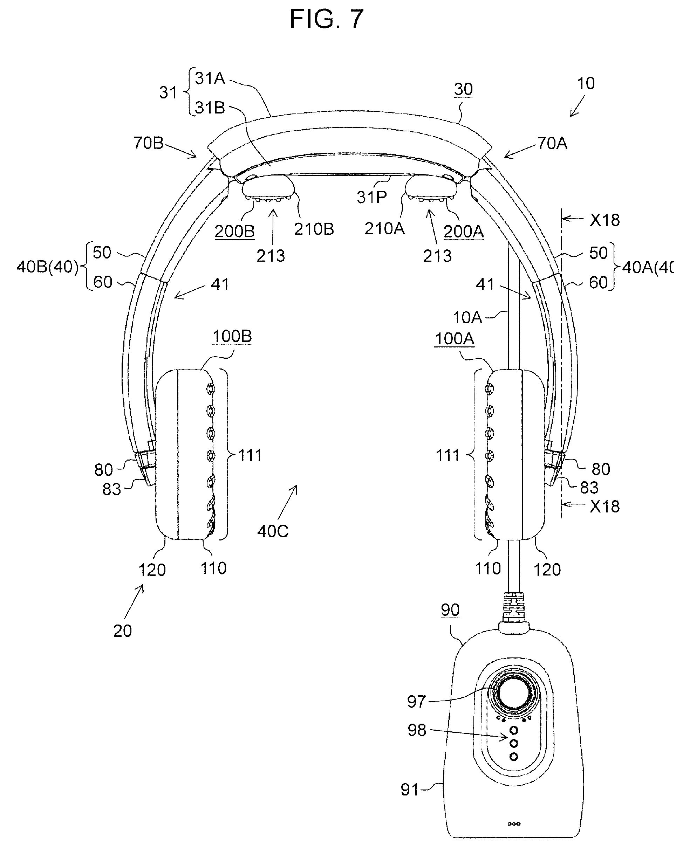

FIG. 7 shows optical hair-growing device 10 of a seventh exemplary embodiment. Optical hair-growing device 10 of the seventh exemplary embodiment further includes the following constitution, which is not described in the sixth exemplary embodiment. Elements given reference numerals common to the sixth exemplary embodiment have similar or analogous functions to the corresponding elements of the sixth exemplary embodiment.

Headset 20 and controller 90 of optical hair-growing device 10 shown in FIG. 7 are one example of a specific form that these objects shown by being modeled in FIG. 6 and the like can take. Headset 20 has a substantially headphone-like shape, and includes a plurality of structural function blocks.

According to the one example, headset 20 includes light emitting unit 30 that outputs light, and a pair of arms 40 that maintains a position of light emitting unit 30 cooperatively, a pair of first attachments 100, and a pair of second attachments 200. First attachments 100 each include first pad 110 that produces soft contact feeling. Second attachments each include second pad 210 that produces soft contact feeling.

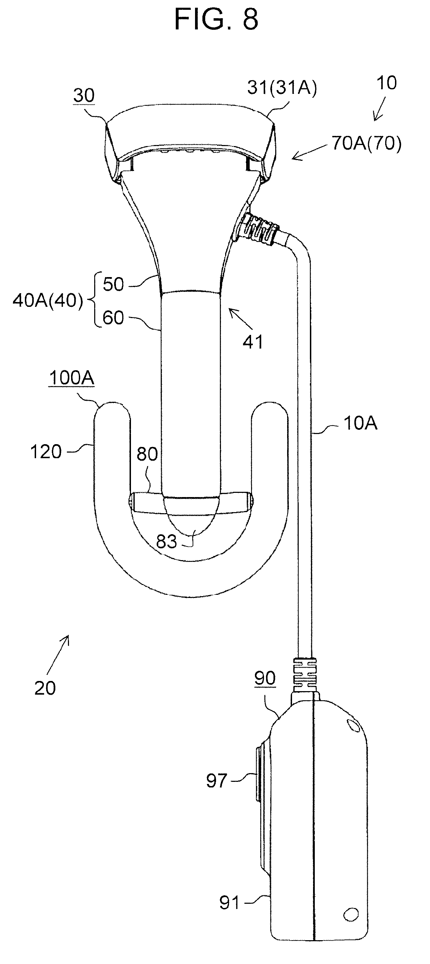

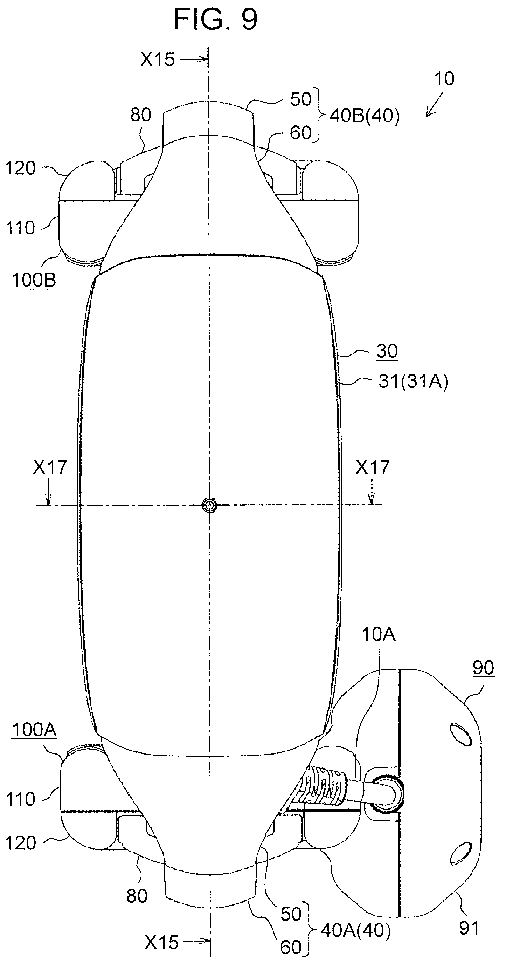

As shown in FIG. 8, cord 10A is drawn to a back surface side of light emitting unit 30. As shown in FIG. 9, first pads 110 protrude to a front side and a rear side with respect to a portion having a largest dimension of light emitting unit 30 in a front-back direction of headset 20. This setting of the dimension contributes to enlargement of an area where each of first pads 110 can come into contact with a side head portion.

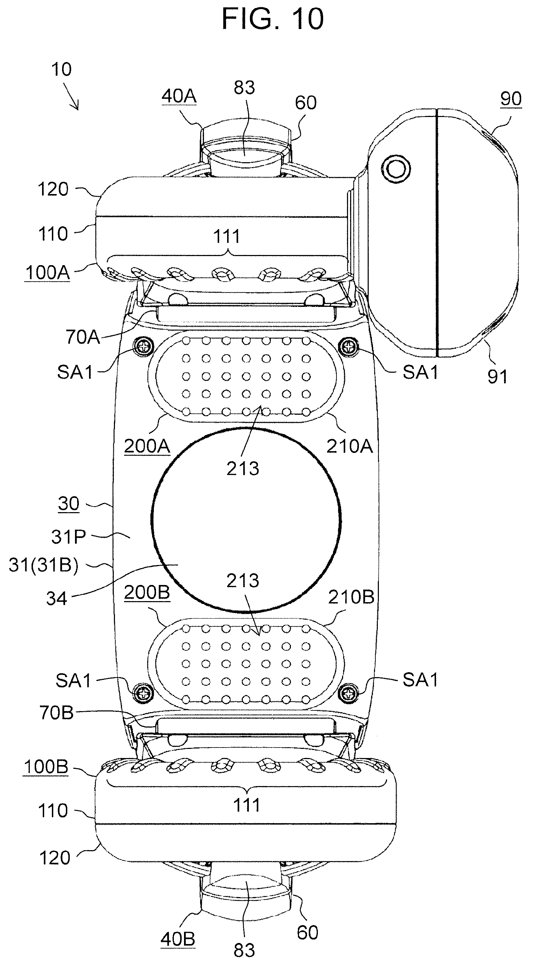

As shown in FIG. 10, left first attachment 100A is arranged outside left second pad 210A in a width direction of headset 20, and inside a portion of arm 40 swelled most outward in the width direction of headset 20. Right first attachment 100B is arranged outside right second pad 210B in the width direction of headset 20, and inside the portion of arm 40 swelled most outward in the width direction of headset 20.

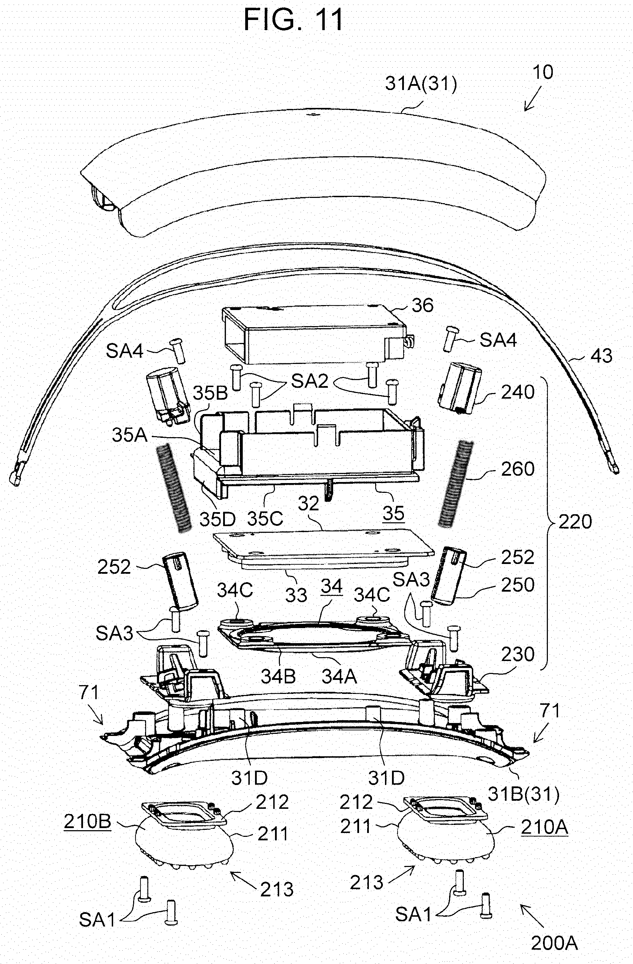

FIG. 11 shows disassembled light emitting unit 30.

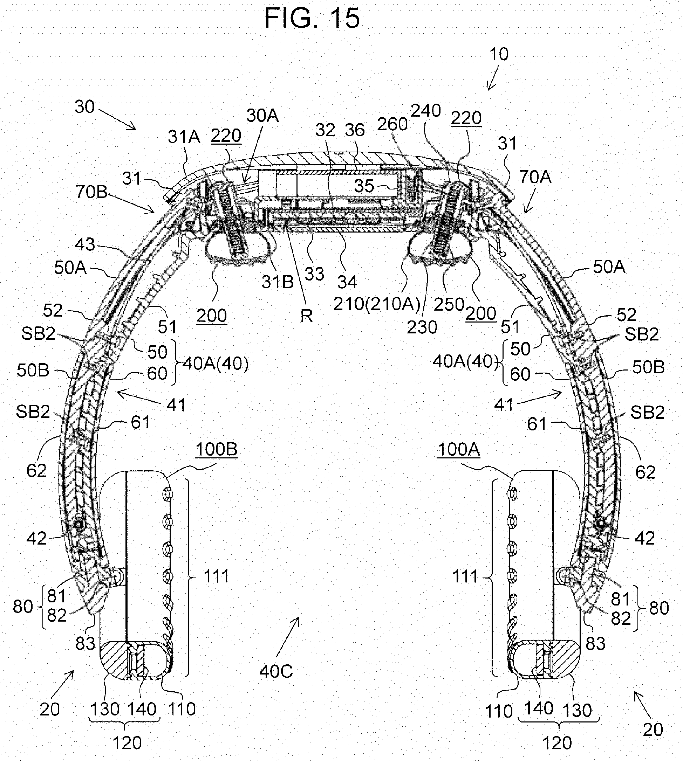

One example of a first reaction-force applying structure included in headset 20 is a plate spring 43 that gives reaction force to respective arms 40. Plate spring 43 is arranged across interiors of unit housing 31 and arms 40, as shown in FIG. 15.

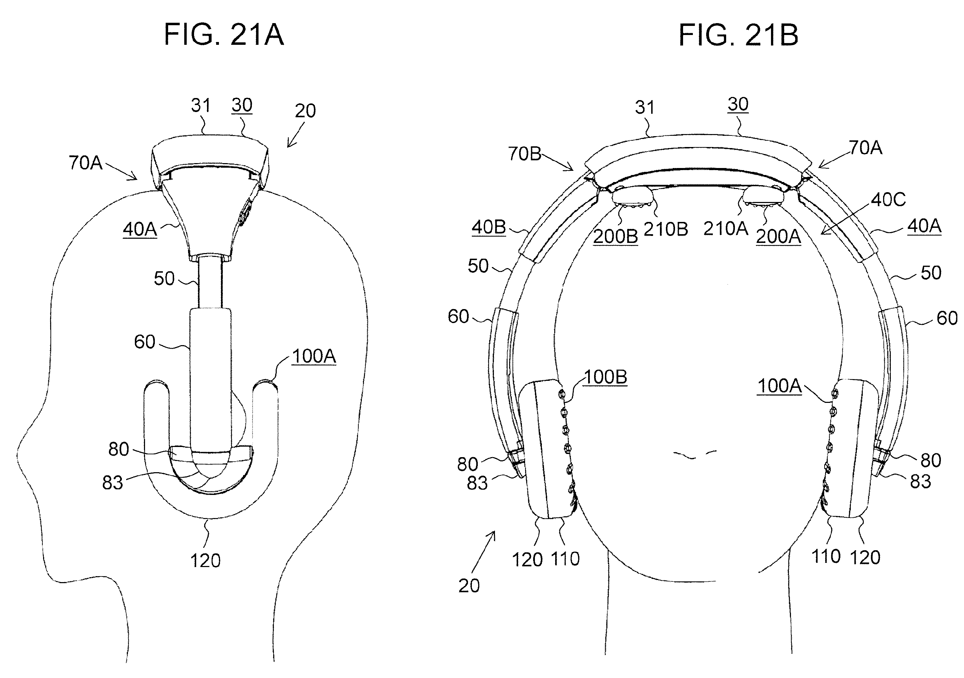

FIGS. 21A and 21B show one example of a state where headset 20 is attached on a head. When headset 20 is attached on the head, a pair of first attachments 100 comes into contact with side head portions, and each of arms 40 rotates about each of hinges 70, so that a distance between the pair of first attachments 100 is increased.

This allows plate spring 43 to produce reaction force to each of arms 40. This reaction force is given to the head through first attachments 100. Therefore, even if force that moves light emitting unit 30 with respect to the head acts, the position of light emitting unit 30 with respect to the head can be maintained by friction force occurring between each of first attachments 100 and the head.

As shown in FIG. 11, light emitting unit 30 is one structural function block that supplies light, and includes a plurality of elements. According to one example, light emitting unit 30 further includes transparent lens 33 that condenses the light output from light source 32, and transparent cover 34 that covers transparent lens 33 in addition to unit housing 31 and light source 32.

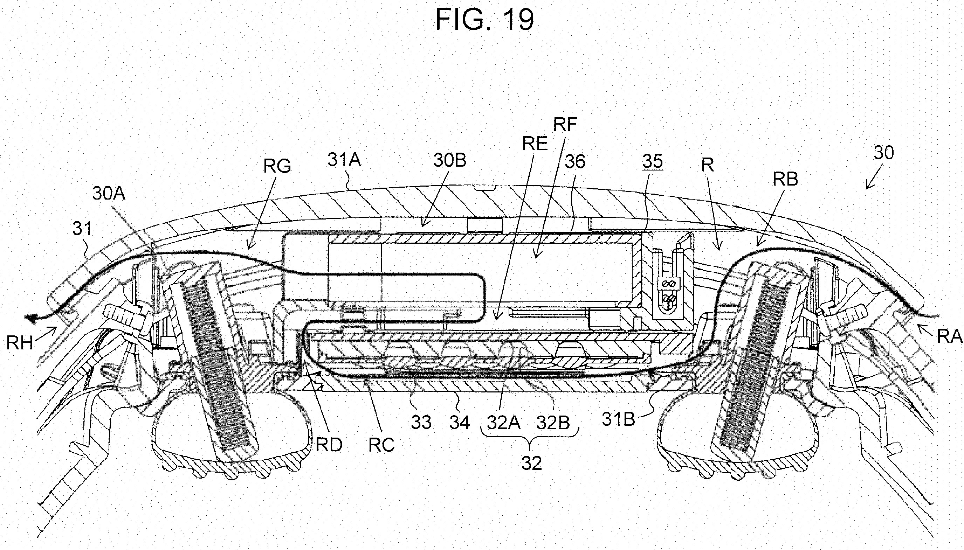

Light emitting unit 30 further includes fan case 35 attached to unit housing 31, electric fan 36 attached to fan case 35, and cooling passage R through which air passes so as to cool light source 32 and the like (refer to FIG. 19).

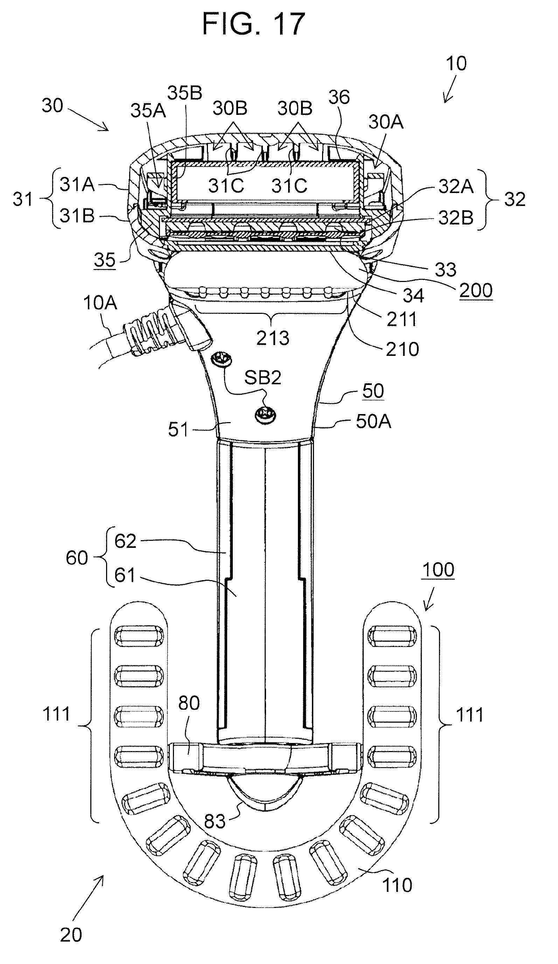

Unit housing 31 is made up of first housing element 31A that forms an upper outer shape of light emitting unit 30, and second housing element 31B that forms a lower outer shape of light emitting unit 30. As shown in FIG. 17, first housing element 31A includes a plurality of support ribs 31C projecting to the interior of unit housing 31.

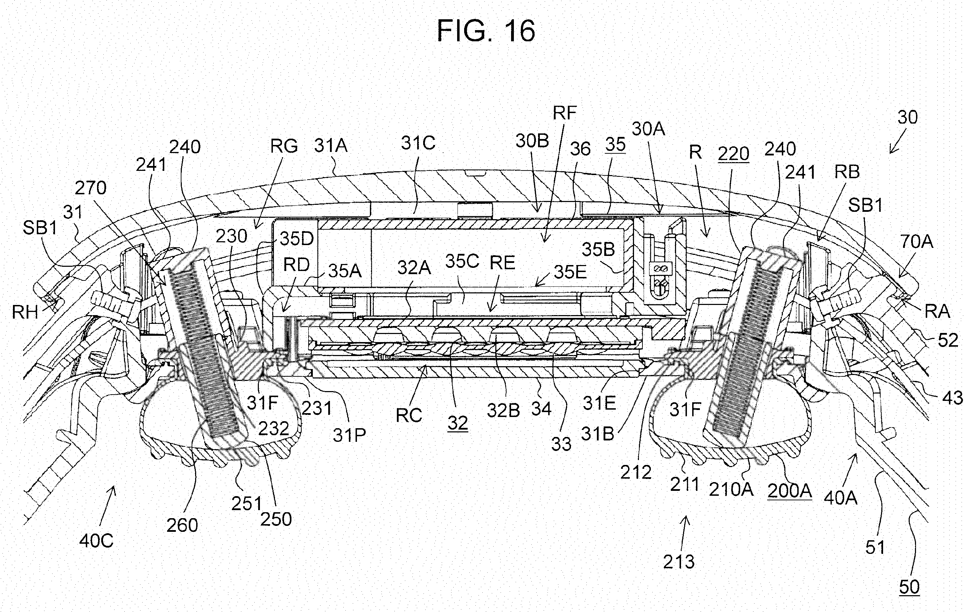

Second housing element 31B includes a plurality of bosses 31D (refer to FIG. 11) inserted into transparent cover 32, cover hole 31E (refer to FIG. 16) into which transparent cover 34 is fitted, and a pair of pad holes 31F (refer to FIG. 16) into which second pads 210 are fitted.

First housing element 31A and second housing element 31B are fixed to each other with four screws SA1 shown in Fig. 11. By fixing first housing element 31A and second housing element 31B to each other, internal space 30A is formed in the interior of unit housing 31, as shown in FIG. 16.

In internal space 30A, the respective elements are stacked and arranged in order of transparent cover 34, transparent lens 33, light source 32, fan case 35, and electric fan 36 from a side of second housing element 31B to a side of first housing element 31A.

As shown in FIG. 16, light source 32 includes substrate 32A to which power supply is supplied from controller 90 through cord 10A (refer to FIG. 8), and a plurality of light emitters 32B mounted on substrate 32A. One example of each of light emitters 32B is an LED that outputs a near-infrared ray. Transparent lens 33 is fixed to substrate 32A so as to cover light emitters 32B.

As shown in FIG. 11, transparent cover 34 includes transmission portion 34A through which the light output from light emitters 32B passes, flange 34B formed around transmission portion 34A, and a plurality of bosses 34C projecting from flange 34B toward a back surface side of transparent cover 34.

Bosses 31D of second housing element 31B are inserted into a plurality of bosses 34C. As shown in FIG. 16, transmission portion 34A is fitted into cover hole 31E of unit housing 31 to form an outer shape of light emitting unit 30 together with opposed surface 31P of unit housing 31.

Joined light source 32 and transparent lens 33 are put on transparent cover 34 so that transparent lens 33 comes into contact with bosses 34C of transparent cover 34. This arrangement allows light-source front-surface passage RC as an air passage to be formed between transmission portion 34A of transparent cover 34 and transparent lens 33, as shown in FIG. 16.

As shown in FIG. 11, fan case 35 includes base 35A that supports electric fan 36, and fan attachment wall 35B that rises from base 35A to a side of first housing element 31A. Fan case 35 further includes light source supporting wall 35C and flow path forming wall 35D that rise from base 35A to a side of the second housing element 31B. Base 35A is formed with vent hole 35E penetrating base 35A.

As shown in FIG. 16, light source supporting wall 35C is put on a back surface of substrate 32A. Flow path forming wall 35D is put on stopper 220, which is one of the elements of second attachment 200. This arrangement allows light-source back-surface passage RE as an air passage to be formed between base 35A and the back surface of substrate 32A, and bent passage RD as an air passage to be formed between flow path forming wall 35D, and substrate 32A and a side surface of transparent lens 33.

Light-source back-surface passage RE includes vent hole 35E formed in base 35A. Bent passage RD has a shape bent so as to go around from a front side of transparent lens 33 to the back surface side of substrate 32A.

Respective four screws SA2 shown in FIG. 11 are inserted into holes of transparent cover 34, transparent lens 33, light source 32, and fan case 35 arranged on second housing element 31B, and screwed into bosses 31D of second housing element 31B. This allows second housing element 31B, transparent cover 34, transparent lens 33, light source 32, and fan case 35 to be fixed to one another.

As shown in FIG. 16, electric fan 36 is fitted inside fan attachment wall 35B of fan case 35, thereby being supported by base 35A. Electric power of controller 90 (refer to FIG. 7) is supplied to electric fan 36 through cord 10A.

An intake port of electric fan 36 connects to vent hole 35E formed in base 35A. An exhaust port of electric fan 36 connects to a portion of internal space 30A of unit housing 31, which portion is formed on a lateral side of fan case 35 and electric fan 36.

As shown in FIG. 17, a plurality of support ribs 31C of first housing element 31A abut on an upper surface of electric fan 36. Upper space 30B, which is a part of internal space 30A, is formed between the upper surface of electric fan 36 and an inner surface of first housing element 31A. Upper space 30B contributes to heat insulation between electric fan 36 and first housing element 31A.

FIG. 11 shows disassembled second attachments 200 and the like.

Each of second attachments 200 is one structural functional block that produces reaction force, based on deformation of second attachment 200 itself, and includes a plurality of elements. According to one example, second attachment 200 further includes stopper 220 that gives the reaction force to second pad 210 in addition to second pad 210. Stopper 220 constitutes a second reaction-force applying structure.

Second pad 210 is formed of a material having elasticity, and one example of the material is silicone rubber or elastomer. As shown in FIG. 10, a dimension in a longitudinal direction of second pad 210 is set to a substantially same size as a diameter of transmission portion 34A of transparent cover 34.

Second pad 210 includes hollow trunk 211 attached to unit housing 31, flange 212 formed around trunk 211, and a plurality of projections 213 formed in an outer surface of trunk 211.

As shown in FIG. 16, trunk 211 is fitted into pad hole 31F of second housing element 31B. Flange 212 is sandwiched between a back surface of second housing element 31B and stopper base 230, which is one element of stopper 220. As shown in FIG. 10, a plurality of projections 213 are formed almost all over the outer surface of trunk 211, which contributes to maintaining of a position of second attachment 200 with respect to the head.

As shown in FIG. 16, stopper 220 includes stopper base 230 fixed to unit housing 31, fixed cover 240 fixed to stopper base 230, and moving cover 250 that moves with respect to stopper base 230. Stopper 220 further includes spring 260 that pushes moving cover 250 to trunk 211 of second pad 210.

As shown in FIG. 16, stopper base 230 includes flange 231 that sandwiches flange 212 of second pad 210 between second housing element 31B and flange 231, and insertion hole 232 that penetrates stopper base 230. Stopper base 230 is fixed to second housing element 31B with two screws SA3 shown in FIG. 11.

As shown in FIG. 16, fixed cover 240 is a tubular object having one end closed by terminal wall 241, and the other end opened, and is fixed to stopper base 230 with one screw SA4 shown in FIG. 11.

As shown in FIG. 16, moving cover 250 is a tubular object having one end closed by terminal wall 251, and the other end opened, and includes projection 252 (refer to FIG. 11) inserted into insertion hole 232 of stopper base 230 to come into contact with stopper base 230.

Terminal wall 251 has a spherical end surface. A portion of moving cover 250 on a side of terminal wall 251 is projected outside stopper base 230 and second housing element 31B and is arranged in an internal space of trunk 211.

Spring 260 is arranged in containing space 270 formed in interiors of fixed cover 240 and moving cover 250, to give moving cover 250 reaction force pushing terminal wall 251 of moving cover 250 to trunk 211.

According to an initial state where no force is applied to trunk 211 from an exterior, force of spring 260 brings projection 252 of moving cover 250 into contact with stopper base 230. This decides a position of moving cover 250 with respect to stopper base 230 and second pad 210. When the initial state is formed, trunk 211 is pushed from an interior to an exterior by terminal wall 251 of moving cover 250, and is deformed so as to swell outward.

Force pushing trunk 211 from the exterior to the interior is applied to trunk 211 from the head, by which moving cover 250 is moved with respect to stopper base 230 and fixed cover 240 to compress spring 260. This allows spring 260 to produce reaction force to moving cover 250. This reaction force is given to the head through trunk 211.

In this manner, second attachment 200 includes a structure that gives the reaction force to the head, based on the deformation of spring 260. The reaction force that second attachment 200 gives to the head is set to a predetermined range suitable for maintaining the position of light emitting unit 30 with respect to the head. One example of the predetermined range is 0.5 N to 5 N. The reaction force that second attachment 200 gives to the head is mainly decided by the reaction force that spring 260 gives to moving cover 250.

Second attachment 200 is further constituted so that by slightly compressing spring 260 from the initial state, that is, when trunk 211 is elastically deformed from the initial state even slightly, the reaction force of a predetermined value or more is generated. One example of the predetermined value is 0.5 N. The numerical values regarding the reaction force of second attachment 200 are examples, and they can be arbitrarily changed in accordance with a weight of light emitting unit 30, or the like.

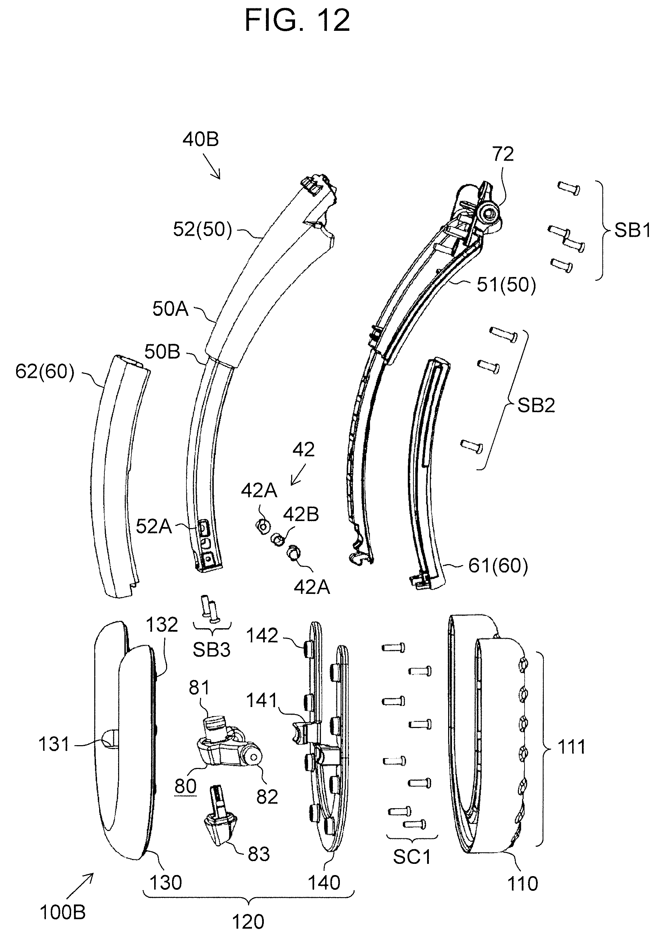

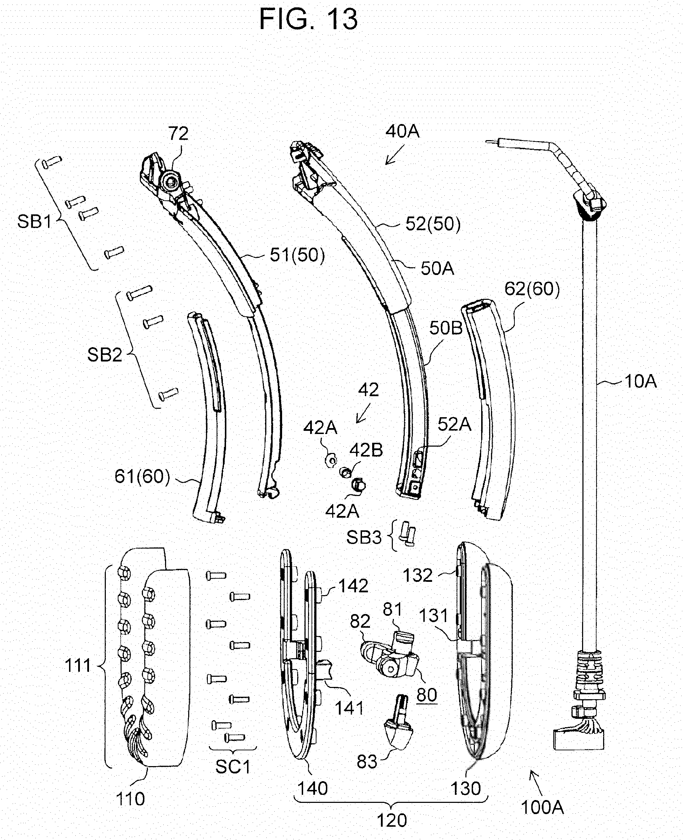

FIG. 12 shows disassembled right arm 40B and the like. FIG. 13 shows disassembled left arm 40A and the like. A pair of arms 40 has a substantially line-symmetrical relationship with respect to a central line in the width direction of headset 20.

Headset 20 further includes a pair of covers 83 attached to joints 80. As shown in FIG. 8, by being attached to joint 80, each of covers 83 is arranged on a terminal side of arm 40 to give a smooth terminal shape to arm 40.

Hinges 70 each include receiver 71 (refer to FIG. 11) formed at an opening of unit housing 31, and boss 72 formed in arm 40 (refer to FIG. 12). These elements are joined relatively rotatably, by which hinge 70 is constituted.

As shown in FIG. 15, left hinge 70A connects between light emitting unit 30 and left arm 40A relatively rotatably. Right hinge 70B connects between light emitting unit 30 and right arm 40B relatively rotatably.

Arms 40 are objects curved so as to swell from an inside to an outside of arrangement space 40C, and each include a plurality of elements. According to one example, each of arms 40 includes an upper arm 50 attached to unit housing 31, and a lower arm 60 attached to upper arm 50.

Arm 40 further includes telescopic structure 41 that relatively slides upper arm 50 and lower arm 60, and lock mechanism 42 that fixes relative positions of upper arm 50 and lower arm 60.

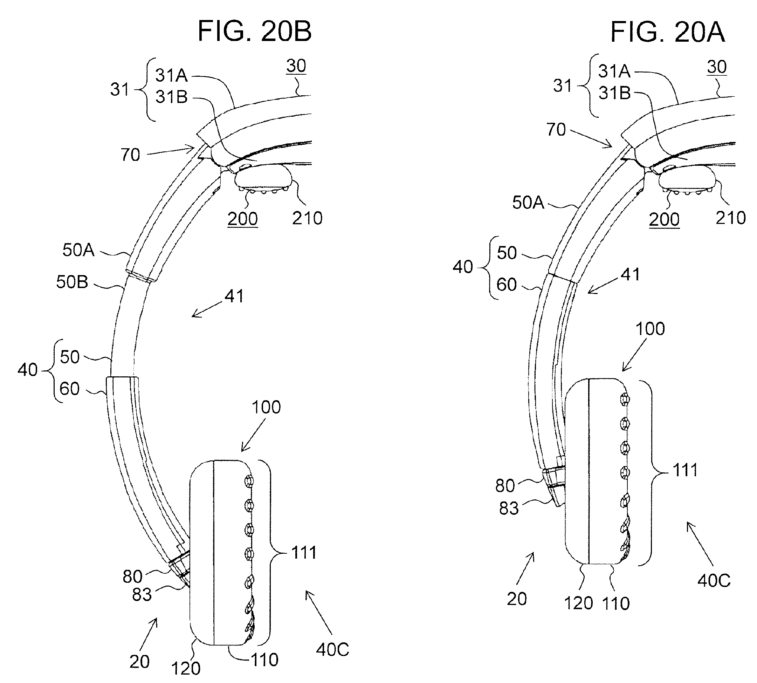

Telescopic structure 41 includes thin portion 50B of upper arm 50, and lower arm 60 attached to thin portion 50B so as to slide against thin portion 50B. A length of arm 40 is changed stepwise in a range of a smallest length shown in FIG. 20A to a largest length shown in FIG. 20B.

As shown in FIG. 15, upper arm 50 includes thick portion 50A containing plate spring 43, and thin portion 50B to which lower arm 60 is attached.

As shown in FIG. 12, upper arm 50 includes first upper element 51 forming an outer shape of an inside of upper arm 50, and second upper element 52 forming an outer shape of an outside of upper arm 50. Boss 72 of hinge 70 is formed at an end of first upper element 51. Second upper element 52 includes containing hole 52A in which lock mechanism 42 is arranged.

Ends of thick portions 50A of first upper element 51 and second upper element 52 are fixed to each other with four screws SB1 shown in FIG. 12. As shown in FIG. 15, thick portions 50A of first upper element 51 and second upper element 52, and plate spring 43 are fixed to each other with one screw SB2.

Thin portions 50B of first upper element 51 and second upper element 52 are fixed to each other with two screw SB2. First lower element 61 and second lower element 62 are fixed to each other with two screws SB3 shown in FIG. 12.

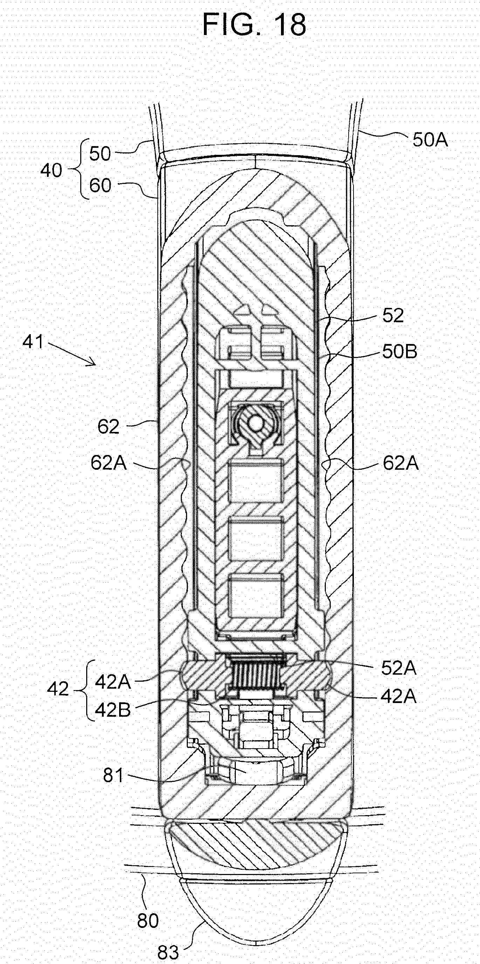

FIG. 18 shows an internal structure of arm 40.

Second lower element 62 of lower arm 60 includes regulation portions 62A in a pair of side surfaces opposed to each other in an interior of second lower element 62. Regulation portions 62A are each a group of a plurality of irregularities formed side by side in a longitudinal direction of the side surface.

Lock mechanism 42 includes a pair of adjustment locks 42A pressed to regulation portions 62A of lower arm 60, and lock spring 42B pressing adjustment locks 42A to regulation portions 62A. Lock spring 42B is arranged in containing hole 52A of upper arm 50. Adjustment locks 42A are each attached to each end of lock spring 42B, and are arranged in containing hole 52A so that terminal portions of adjustment locks 42A are projected from containing hole 52A.

Depressions of regulation portion 62A each have a shape corresponding to a shape of the terminal of adjustment lock 42A. A range where upper arm 50 and lower arm 60 can relatively slide, that is, a range where the length of arm 40 is changed is decided by a range in the longitudinal direction where regulation portions 62A are formed in lower arm 60.

Adjustment locks 42A are pressed to the depressions of regulation portions 62A, by which adjustment locks 42A and regulation portions 62A regulate relative sliding between upper arm 50 and lower arm 60 cooperatively. This maintains the length of arm 40.

Force maintaining the state where adjustment locks 42A are pressed to the depressions of regulation portions 62A, that is, maintaining force of lock mechanism 42, which is force maintaining the length of arm 40, is one of major elements that decide stability of headset 20 with respect to the head.

The maintaining force of lock mechanism 42 is mainly decided by friction force occurring between adjustment locks 42A and regulation portions 62A. This friction force is mainly decided by reaction force that lock spring 42B gives to adjustment locks 42A.

When force acting in a direction where upper arm 50 and lower arm 60 are relatively slid does not act on arm 40, the length of arm 40 is maintained by the maintaining force of lock mechanism 42.

When the above-described force acts on arm 40, force that displaces adjustment locks 42A to a side of containing hole 52A against the reaction force of lock spring 42B acts on adjustment locks 42A. However, if the force is smaller than the maintaining force of lock mechanism 42, the length of arm 40 is still maintained by the maintaining force of lock mechanism 42.

When the force that displaces adjustment locks 42A to the side of containing hole 52A is larger than the maintaining force of lock mechanism 42, adjustment locks 42A are withdrawn from the depressions of regulation portions 62A, so that upper arm 50 and lower arm 60 relatively slide. This changes the length of arm 40.

As shown in FIGS. 21A and 21B, when headset 20 is attached on the head, the displacement promoting force acts on each of second attachments 200, based on the reaction force that second attachment 200 gives to the head. This displacement promoting force acts so as to displace moving cover 250 to an opposite side of arrangement space 40C through trunk 211 of second pad 210 shown in FIG. 16, and further acts on arm 40 through stopper base 230 and unit housing 31.

When headset 20 is attached on the head, a pair of first attachments 100 is in contact with the head, as shown in FIGS. 21A and 21B.

Therefore, by the displacement promoting force acting on arm 40 and the friction force occurring between first attachment 100 and the head, the extension promoting force, which is force that slides upper arm 50 with respect to lower arm 60, acts on arm 40. As a result, the force that is to move adjustment locks 42A shown in FIG. 18 to the side of containing hole 52A acts.

The maintaining force of lock mechanism 42 is set larger than the above-described force acting on the adjustment locks 42A, based on the extension promoting force. This allows lock mechanism 42 to regulate the relative sliding between upper arm 50 and lower arm 60, and the length of arm 40 to be maintained, even when the displacement promoting force acts on second attachment 200.

According to one example, the reaction force given from lock spring 42B (refer to FIG. 18) to adjustment locks 42A is set larger than the reaction force given from spring 260 (refer to FIG. 16) to the head, by which the above-described maintaining force of lock mechanism 42 can be obtained.

FIGS. 12 and 13 show disassembled first attachments 100.

First attachments 100 are each one structural functional block that produces reaction force, and each include a plurality of elements. According to one example, each of first attachments 100 further includes holder 120 connected to arm 40 by joint 80 in addition to first pad 110.

Holder 120 includes curved case 130 that forms an outer shape of first attachment 100, and curved cover 140 attached to curved case 130. Curved case 130 and curved cover 140 have similar U-shapes, and are fixed to each other with eight screws SC1.

Curved case 130 includes depressions 131 where second shaft 82 of joint 80 is arranged, and a plurality of bosses 132 into which screws SC1 are screwed. Curved cover 140 includes bearing 141 that supports second shaft 82 of joint 80, and a plurality of bosses 142 into which screws SC1 are inserted.

Joint 80 includes first shaft 81 attached to arm 40, and second shaft 82 attached to holder 120. First shaft 81 has a first rotation axis extending in a substantially same direction as the longitudinal direction of arm 40, and is sandwiched between first lower element 61 and second lower element 62 rotatably with respect to arm 40. The longitudinal direction of arm 40 is defined, for example, by a tangent of arm 40 in the portion where arm 40 swells most outward.

Second shaft 82 has a second rotation axis extending in a substantially same direction as a width direction of holder 120, and is sandwiched between curved case 130 and curved cover 140 rotatably with respect to holder 120. The width direction of holder 120 is a direction perpendicular to the longitudinal direction of arm 40 in front view of first attachment 100 shown in FIG. 17.

As shown in FIG. 15, the direction where the first rotation axis extends is the same or almost the same direction as a height direction of headset 2. The direction where the second rotation axis extends is the same or almost the same as a front-back direction of headset 20.

First attachment 100 is attached to joint 80 so as to be able to rotate about second shaft 82 with respect to joint 80, and so as not to rotate about first shaft 81 with respect to joint 80.

Joint 80 rotates about first shaft 81 with respect to arm 40, by which a posture of first attachment 100 with respect to arm 40 is changed. First attachment 100 can take both of a posture in which a front portion of first attachment 100 comes into arrangement space 40C, and a posture in which the front portion of first attachment 100 protrudes from arrangement space 40C.

First attachment 100 rotates about second shaft 82 with respect to joint 80, by which the posture of first attachment 100 with respect to arm 40 is changed. First attachment 100 can take both of a posture in which a terminal portion of first attachment 100 comes into arrangement space 40C and a posture in which the terminal portion of first attachment 100 protrudes from arrangement space 40C.

FIGS. 12 and 13 show first pad 110 separated from holder 120.

First pad 110 is formed of a material having elasticity, and one example of the material is silicon rubber or elastomer.

First pad 110 curves in a U shape similar to holder 120 and includes a plurality of projections 111. The plurality of projections 111 contribute to maintaining of a position of first attachment 100 with respect to the head. According to one example, a size of each of projections 111 is set larger than each of projections 213 of second pad 210.

Maintaining force of first attachment 100, which is force by which first attachment 100 maintains its own position with respect to the head, is one of major elements that decide the stability of headset 20 with respect to the head. The maintaining force of first attachment 100 is mainly decided by friction force occurring between first pad 110 and the head. This friction force is mainly decided by the reaction force that plate spring 43 gives to arm 40.

As shown in FIGS. 21A and 21B, when headset 20 is attached on the head, the displacement promoting force acts on each of second attachments 200, based on the reaction force that second attachment 200 gives to the head. This displacement promoting force acts on each of first attachments 100 through moving cover 250, stopper base 230, unit housing 31, and arm 40.

Therefore, force that is to move first attachment 100 with respect to the head acts on first attachment 100.

The maintaining force of first attachment 100 is set larger than force that is to move first attachment 100 with respect to the head, based on the displacement promoting force. Thus, even when the displacement promoting force acts on second attachment 200, first attachment 100 substantially does not move with respect to the head, and the positions of arm 40 and light emitting unit 30 with respect to the head are maintained.

According to one example, the reaction force given to each of arms 40 by plate spring 43 (refer to FIG. 15) is set larger than the reaction force given to the head by spring 260 (refer to FIG. 16), by which the above-described maintaining force of first attachment 100 can be obtained.

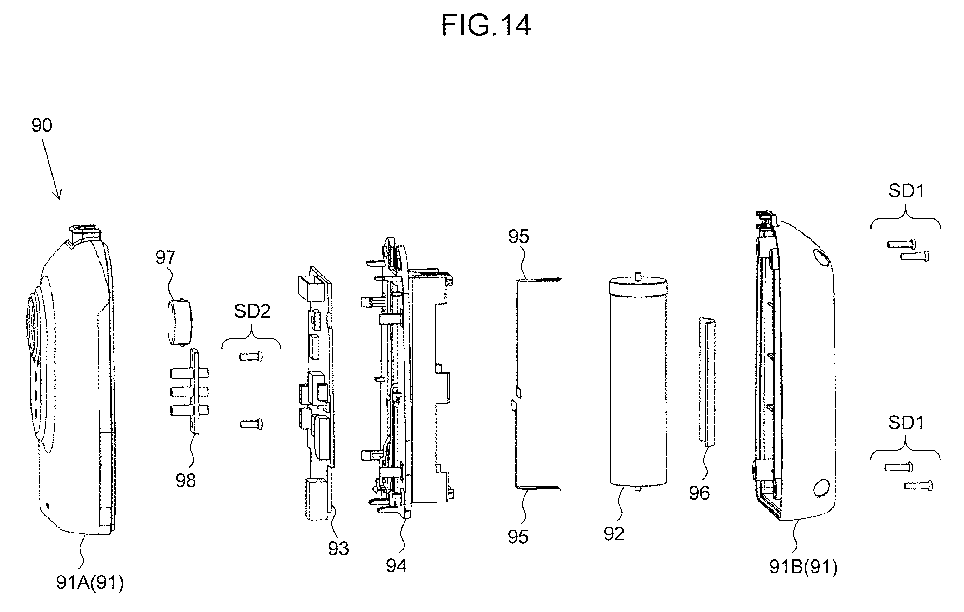

FIG. 14 shows disassembled controller 90.

Controller 90 has enough size to be held by hand, and includes a plurality of elements. According to one example, in addition to case 91, a battery as power supply 92, and circuit board 93, controller 90 includes base 94 to which circuit board 93 and power supply 92 are attached, and a pair of electrode fittings 95 connected to power supply 92.

Controller 90 further includes battery pressing member 96 that presses power supply 92 to base 94, switch button 97 exposed from case 91, and lamp cover 98 that transmits light output from light emitters.

Case 91 is made up of first case element 91A forming an outer shape of a front side of case 91, and second case element 91B forming an outer shape of a back surface side of case 91. First case element 91A, second case element 91B, and base 94 are fixed to each other with four screws SD1. Lamp cover 98 is fixed to a back surface of first case element 91A with two screws SD2.

On circuit board 93 are mounted the plurality of light emitters that are lighted or put out in accordance with operation of light emitting unit 30, and a substrate switch that is set on, accompanying the pressing of switch button 97. A pair of electrode fittings 95 is fixed to base 94. Battery pressing member 96 is attached to a back surface of second case element 91B.

FIG. 16 shows an internal structure of light emitting unit 30.

Cooling passage R of light emitting unit 30 is formed among the plurality of elements making up light emitting unit 30 to mainly contribute to cooling of light source 32, transparent lens 33, and transparent cover 34. According to one example, cooling passage R includes passage entrance RA, fan upstream passage RB, light-source front-surface passage RC, bent passage RD, light-source back-surface passage RE, fan internal passage RF, fan downstream passage RG, and passage exit RH.

Passage entrance RA is a clearance formed between one opening end of unit housing 31 and one of arms 40, and connects to an external space of unit housing 31. According to the illustrated example, a clearance between first housing element 31A and the one of arms 40 forms passage entrance RA.

Fan upstream passage RB is a space around one of stoppers 220 in internal space 30A of unit housing 31, and connects to a downstream portion of passage entrance RA. Light-source front-surface passage RC is a passage formed between a front surface of transparent lens 33 and a back surface of transparent cover 34, and connects to a downstream portion of fan upstream passage RB.

Bent passage RD is a passage formed between a side surface of light source 32 and transparent lens 33, and flow path forming wall 35D of fan case 35, and bent into a U shape, and connects to a downstream portion of light-source front-surface passage RC. The light-source back-surface passage RE is a passage including a passage formed between a back surface of substrate 32A of light source 32 and base 35A of fan case 35, and vent hole 35E of fan case 35, and connects to a downstream portion of bent passage RD.

Fan internal passage RF is a passage formed in an interior of electric fan 36, and connects to vent hole 35E, which is a downstream portion of light-source back-surface passage RE. Fan downstream passage RG is a space around the other one of stoppers 220 in internal space 30A of unit housing 31, and connects to an exit of electric fan 36, which is a downstream portion of fan internal passage RF.

Passage exit RH is a clearance formed between the other opening end of unit housing 31 and the other one of arms 40, and connects to a downstream portion of fan downstream passage RG and an external space of unit housing 31. According to the illustrated example, a clearance between first housing element 31A and the other one of arms 40 forms passage exit RH.

FIG. 19 shows an air flow formed by electric fan 36.

When controller 90 (refer to FIG. 7) is operated and electric fan 36 starts to rotate, air existing in fan internal passage RF is discharged to fan downstream passage RG, and in addition, air existing in light-source back-surface passage RE is sucked into fan internal passage RF. The air discharged to fan downstream passage RG passes passage exit RH, and flows out to the exterior of unit housing 31.

Accompanying the suction of the air existing in light-source back-surface passage RE into fan internal passage RF, air existing in bent passage RD flows into light-source back-surface passage RE, and air existing in light-source front-surface passage RC flows into bent passage RD. Furthermore, air existing in fan upstream passage RB flows into light-source front-surface passage RC, and air existing in the exterior of unit housing 31 passes passage entrance RA and flows into fan upstream passage RB.

In this manner, the rotation of electric fan 36 allows the air in the exterior of unit housing 31 to flow into unit housing 31 from passage entrance RA.

The air that has passed passage entrance RA flows through cooling passage R in order of fan upstream passage RB, light-source front-surface passage RC, bent passage RD, light-source back-surface passage RE, fan internal passage RF, and fan downstream passage RG, and is exhausted to the exterior of unit housing 31 through passage exit RH. The air flowing cooling passage R cools transparent cover 34, transparent lens 33, and light source 32.

Optical hair-growing device 10 is used, for example, as follows.

As shown in FIGS. 21A and 21B, the head is arranged in arrangement space 40C of headset 20, and headset 20 is attached on the head, by which, for example, the following state is formed. Light emitting unit 30 is arranged on a head top portion, which is one example of an object site of the head.

A pair of first pads 110 comes into contact with side head portions so as to cover ears. Accompanying the contact between first pads 110 and the side head portions, arms 40 rotate about hinges 70, so that a distance between the pair of first pads 110 is increased.

With the rotation of arms 40, plate spring 43 (refer to FIG. 15) gives the reaction force to arms 40. This reaction force is given to the head through first attachments 100. This allows the pair of first attachments 100 to sandwich the head cooperatively. A pair of second attachments 200 each comes into contact with the head top portion. The contact between second attachments 200 and the head top portion allows springs 260 (refer to FIG. 16) to give the reaction force to the head.

When a user operates controller 90 after attaching headset 20 on the head, the operation of light source 32 and electric fan 36 starts. The light output from light source 32 is supplied to the object site of the head. This may bring about the effect of promoting the hair growing.

According to optical hair-growing device 10 of the seventh exemplary embodiment, the following effects can be obtained in addition to the effects of (1) to (8) obtained by optical hair-growing device 10 of the sixth exemplary embodiment.
(9) First attachments 100 each include elastically deformable first pad 110. According to this constitution, even when headset 20 is attached on each of the heads having various shapes, first pads 110 are elastically deformed in accordance with the head to properly give the reaction force to the head. This enables first pads 110 to firmly hold the head even when headset 20 is attached on each of the heads having various shapes. Therefore, the position of light emitting unit 30 with respect to the head is hard to be displaced.
(10) Second attachments 200 each include elastically deformable second pad 210. According to this constitution, even when headset 20 is attached on each of the heads having various shapes, second pads 210 are elastically deformed in accordance with the head to properly give the reaction force to the head. This enables second pads 210 to firmly hold the head even when headset 20 is attached on each of the heads having various shapes. Therefore, the position of light emitting unit 30 with respect to the head is hard to be displaced.
(11) When optical hair-growing device 10 is attached on the head, in some shapes of the head, a pair of first attachments 100 comes into firm contact with the head, while second attachments 200 may be hard to come into firm contact with the head. Therefore, according to a different form that optical hair-growing device 10 can take, that is, a form where the constitution shown in the seventh exemplary embodiment is not included in second attachments 200, there is a possibility that second attachments 200 hinder the effect of maintaining the position of light emitting unit 30 with respect to the head from being obtained.
   In order to solve or alleviate the above-described problem, optical hair-growing device 10 includes the constitution where when second pads 210 are even slightly elastically deformed, the reaction force of the predetermined value or more is generated. According to this constitution, even if the shape of the head is hard to come into firm contact with second attachments 200, if second pads 210 come into enough contact with the head to slightly elastically deform second pads 210, the second attachments 200 generate the reaction force of the predetermined value or more
   Second attachments 200 substantially contribute to suppression of displacement of light emitting unit 30 with respect to the head when the reaction force of the predetermined value or more is generated. That is, the contact of second pads 210 with the head allows the role of second attachments 200 to be substantially played. This enables optical hair-growing device 10 to firmly maintain the position of light emitting unit 30 with respect to the head, even when optical hair-growing device 10 is attached on each of the heads having various shapes.
(12) Headset 20 includes electric fan 36 and cooling passage R. According to this constitution, the air flowing in cooling passage R cools transparent cover 34, transparent lens 33, and light source 32. Thus, even if headset 20 has been continuously operated for a long time, transparent cover 34, transparent lens 33, and light source 32 are hard to have a high temperature.

### (Modifications)

Descriptions of the respective exemplary embodiments are examples of the exemplary embodiments that the optical hair-growing device of the present disclosure can take, and are not intended to limit the exemplary embodiments. The optical hair-growing device of the present disclosure can take modifications of the respective exemplary embodiments, for example, as described below beside the respective exemplary embodiments.
- Headset 20 of a modification includes springs that press lower arms 60 in a direction where arms 40 extend, and a structure to maintain the length of arms 40 against force of the springs in place of adjustment locks 42A and lock springs 42B.
- Each of second attachments 200 of a modification does not include stopper 220, and gives only the reaction force based on the elastic deformation of second pad 210 to the head. A magnitude of the reaction force that second pad 210 gives to the head can be adjusted, for example, in accordance with a thickness of trunk 211.
- Each of second attachments 200 of a modification includes a sheet-like second pad attached to opposed surface 31P of unit housing 31 in place of second pad 210. This second pad also gives the reaction force to the head by being elastically deformed similarly to second pad 210 of the respective exemplary embodiments.
- Each of hinges 70 of a modification includes a rotation axis extending in a direction intersecting the front-back direction of headset 20 in place of the rotation axis defined by receiver 71 and boss 72.
- Each of joints 80 of a modification includes only one of first shaft 81 and second shaft 82.
- Each of joints 80 of a modification includes a first rotation axis extending in a substantially same direction as the vertical direction of headset 20 in place of the first rotation axis defined by first shaft 81.
- Each of joints 80 of a modification includes a second rotation axis extending in a direction intersecting the front-back direction of headset 20 in place of the second rotation axis defined by second shaft 82.
- Headset 20 of a modification includes a structure in which light emitting unit 30 and a pair of arms 40 are integrated. According to one example of the structure, unit housing 31 of light emitting element 30 and each of arms 40 are integrally formed of the same material.
- Headset 20 of a modification includes, for example, a following first reaction-force applying structure in place of the first reaction-force applying structure including plate spring 43. A first modification regarding the first reaction-force applying structure includes a spring arranged in an interior of each of first attachments 100 (illustration is omitted).

This reaction-force applying structure gives the reaction force to the head by deformation of the spring caused by attaching headset 20 on the head, and this reaction force maintains the position of first attachment 100 with respect to the head.

A second modification regarding the first reaction-force applying structure includes a different first pad that produces reaction force stronger than first pad 110 in place of first pad 110 of the exemplary embodiments. This reaction-force applying structure gives the reaction force to the head by elastic deformation of the different first pad caused by attaching the headset 20 on the head, and maintains the position of first attachment 100 with respect to the head by the reaction force.
- Headset 20 of a modification includes, for example a following second reaction-force applying structure in place of the second reaction-force applying structure including stopper 220 arranged in the interior of each of second attachments 200. A first modification regarding the second reaction-force applying structure includes a spring (illustration is omitted) arranged in the interior of light emitting unit 30.

This reaction-force applying structure gives the reaction force to the head through each of second attachments 200 by the deformation of the spring caused by attaching headset 20 on the head, and maintains the position of second attachment 200 by this reaction force.

A second modification regarding the second reaction-force applying structure includes a different second pad that produces reaction force stronger than second pad 210 in place of second pad 210 of the exemplary embodiments. This reaction-force applying structure gives the reaction force to the head by elastic deformation of the different second pad caused by attaching headset 20 on the head, and maintains the position of second attachment 200 by this reaction force.
- Headset 20 of a modification includes only one second attachment 200, or includes three or more second attachments 200.
- Headset 20 of a modification includes a power supply or a power supply circuit. The power supply or the power supply circuit is arranged, for example, in the interior of light emitting unit 30.

The above detailed description is intended to be illustrative, and not restrictive. For example, the above-described respective exemplary embodiments or one or more modifications leave room for mutual combination as needed.

Technical characteristics or a subject of the present disclosure can exist in fewer characteristics than all the characteristics of the specific exemplary embodiment. Thus, the scope of claims is incorporated in the detailed description of the invention, and each of the claims claims itself as the individual exemplary embodiment.

### (Supplementary Notes Regarding Means for Solving Problems)

[Supplementary note 1]: An optical hair-growing device includes a light emitting unit that outputs light, and the light emitting unit includes a light source that outputs the light, an electric fan that discharges air, a fan case that supports the electric fan, and a cooling passage that cools the light source, and the cooling passage includes a bent passage bent so as to go around from a front surface of the light source to a back surface of the light source.
[Supplementary note 2]: In the optical hair-growing device according to Supplementary note 1, the fan case includes a base that supports the electric fan, and a flow path forming wall that rises from the base to a light source side, and the bent passage is formed between the flow path forming wall and the light source.
[Supplementary note 3]: The optical hair-growing device according to Supplementary note 2 further includes a pair of arms attached to the light emitting unit, and the light emitting unit includes a unit housing that contains the light source, the electric fan, and the fan case. One of the arms is connected to one opening end of the unit housing, the other arm is connected to the other opening end of the unit housing, a clearance formed between the one arm and the unit housing constitutes an entrance of the cooling passage, and a clearance formed between the other arm and the unit housing constitutes an exit of the cooling passage.
[Supplementary note 4]: In the optical hair growing device according to any one of Supplementary notes 1 to 3, the light emitting unit further includes a transparent lens attached to the light source, and a transparent cover that protects the transparent lens, the cooling passage further includes a light-source front-surface passage formed between a back surface of the transparent cover and the transparent lens, and a light-source back-surface passage formed between a back surface of the light source and the fan case, and the light-source front-surface passage and the light-source back-surface passage are connected by the bent passage.

As described above, the present disclosure can be applied to an optical hair-growing device for household or business. The present disclosure can also be applied to other electrical instruments used by being attached on a head like a headphone or the like.

## Claims

1. An optical hair-growing device (10) comprising:
a light emitting unit (30) configured to output light;
at least one arm (40) attached to the light-emitting unit (30);
a pair of first attachments (100) attached to the arm (40) to produce reaction force;
at least one second attachment (200) attached to the light emitting unit (30) or the arm (40) to produce reaction force;
a pair of joints (80) that connects the arm (40) and the first attachments (100); and
a structure in which each of the first attachments is rotatable with respect to each of the joints about a rotation axis extending in the same direction or in substantially the same direction as a lateral direction of the arm;
**characterized in that**
the optical hair-growing device (10) further comprises a structure in which each of the joints (80) is rotatable with respect to the arm (40) about a rotation axis extending in the same direction or in substantially the same direction as a longitudinal direction of the arm (40).

2. The optical hair-growing device (10) according to claim 1, wherein
the second attachment (200) is attached to the light emitting unit (30) or the arm (40) so as not to disturb travel of the light output from the light emitting unit (30).

3. The optical hair-growing device (10) according to claim 1, wherein
a pair of arms (40) is included,
the pair of arms (40) includes a left arm (40A) and a right arm (40B),
one of the first attachments (100A) is attached to the left arm (40A),
the other first attachment (100B) is attached to the right arm (40B),
a left hinge (70A) connecting the left arm (40A) and the light emitting unit (30), and a right hinge (70B) connecting the right arm (40B) and the light emitting unit (30) are further included,
the left hinge (70A) being configured to rotate the left arm (40A) with respect to the light emitting unit (30) so as to change a distance between the one first attachment (100A) and the other first attachment (100B), and
the right hinge (70B) being configured to rotate the right arm (40B) with respect to the light emitting unit (30) so as to change the distance between the one first attachment (100A) and the other first attachment (100B).

4. The optical hair-growing device (10) according to claim 1, wherein
the second attachment (200) is configured to generate reaction force of a predetermined value or more upon elastic deformation of the second attachment (200).

5. The optical hair-growing device (10) according to claim 1, wherein
the second attachment (200) is attached to the light emitting unit (30), and
force with which the first attachments (100) maintain their own positions is set larger than force that is to move the first attachments (100), based on the reaction force that the second attachment (200) produces.

6. The optical hair-growing device (10) according to claim 1, wherein
the arm (40) further comprises a telescopic structure configured to change a length of the configured to maintain a arm (40) itself, and a lock mechanism (42) configured to maintain the length of the arm (40),
and
force generated by the lock mechanism (42) to maintain the length of the arm (40) is set larger than force that extends the arm (40), based on the reaction force that the second attachment (200) produces.

## Patentansprüche

1. Optische Haarwachsvorrichtung (10) umfasst:
eine lichtemittierende Einheit (30) konfiguriert ist um Licht auszugeben;
mindestens einen Arm (40), der an der lichtemittierenden Einheit (30) angebracht ist;
ein Paar von ersten Befestigungen (100), die an dem Arm (40) angebracht sind um Auflagekraft zu erzeugen;
mindestens eine zweite Befestigung (200), die an der Lichtaustritteinheit (30) oder dem Arm (40) angebracht ist um Auflagekraft zu erzeugen;
ein Paar von Gelenken (80), die den Arm (40) und die ersten Befestigungen (100) verbinden; und
eine Struktur in welcher jeder der ersten Befestigungen zu jedem der Gelenken um eine Rotationsachse in die gleiche Richtung verlängernd oder im Wesentlichen in gleiche Richtung wie eine laterale Richtung des Arms, rotierbar ist;
**dadurch gekennzeichnet, dass**
die optische Haarwachsvorrichtung (10) des Weiteren eine Struktur umfasst, in welcher jedes der Gelenken (80) zu dem Arm (40) um eine Rotationsachse in die gleiche Richtung verlängernd oder im Wesentlichen in gleiche Richtung wie eine longitudinale Richtung des Arms (40), rotierbar ist.

2. Die optische Haarwachsvorrichtung (10) nach Anspruch 1, wobei die zweite Befestigung (200) an der lichtemittierenden Einheit (30) oder dem Arm (40) befestigt ist, um den Weg des Lichtaustritts von der lichtemittierenden Einheit (30) nicht zu stören.

3. Die optische Haarwachsvorrichtung (10) nach Anspruch 1, wobei
ein Paar von Armen (40) enthalten ist,
die Paar von Armen (40) enthalten einen linken Arm (40A) und einen rechten Arm (40B),
ein der ersten Befestigungen (100A) ist an dem linken Arm (40A) angebracht,
die andere erste Befestigung (100B) ist an dem rechten Arm (40B) angebracht,
ein linkes Gelenk (70A), das den linken Arm (40A) und die lichtemittierende Einheit (30) verbindet, und ein rechtes Gelenk (70B), das den rechten Arm (40B) und die lichtemittierende Einheit (30) verbindet, sind des Weiteren enthalten,
das linke Gelenk (70A) wird konfiguriert, um den linken Arm (40A) in Bezug auf die lichtemittierende Einheit (30) zu rotieren, um den Abstand zwischen der einen ersten Befestigung (100A) und der anderen zweiten Befestigung (100B) zu ändern, und das rechte Gelenk (70B) wird konfiguriert, um den rechten Arm (40B) in Bezug auf die lichtemittierende Einheit (30) zu rotieren, um den Abstand zwischen der einen ersten Befestigung (100A) und der anderen zweiten Befestigung (100B) zu verändern.

4. Die optische Haarwachsvorrichtung (10) nach Anspruch 1, wobei die zweite Befestigung (200) konfiguriert ist, um Auflagekraft eines vorgegebenen Wertes oder mehr an elastischer Verformung der zweiten Befestigung (200), zu erzeugen.

5. Die optische Haarwachsvorrichtung (10) nach Anspruch 1, wobei die zweite Befestigung (200) an der lichtemittierenden Einheit (30) angebracht ist, und Kraft mit welcher die ersten Befestigungen (100) ihre eigene Position aufrechterhalten, ist größer festgelegt als die Kraft, die zum Bewegen der ersten Befestigungen (100), ausgehend von der Auflagekraft, die die zweite Befestigung (200) erzeugt.

6. Die optische Haarwachsvorrichtung (10) nach Anspruch 1, wobei der Arm (40) des Weiteren eine Teleskopstruktur aufweist, die konfiguriert ist um eine Länge des Arms (40) selbst zu verändern, und ein Sperrmechanismus (42), der konfiguriert ist um die Länge des Arms (40) aufrechtzuerhalten, und Kraft, die durch das Sperrmechanismus (42) generiert wird um die Länge des Armes (40) aufrechtzuerhalten, größer festgelegt ist als die Kraft die den Arm (40) verlängert, ausgehend von der Auflagekraft, die die zweite Befestigung (200) erzeugt.

## Revendications

1. Dispositif optique de croissance pileuse (10) comprenant :
une unité d'émission de lumière (30) configurée pour délivrer en sortie de la lumière,
au moins un bras (40) fixé à l'unité d'émission de lumière (30),
une paire de premiers accessoires (100) fixés au bras (40) afin de produire une force de réaction,
au moins un second accessoire (200) fixé à l'unité d'émission de lumière (30) ou au bras (40) afin de produire une force de réaction,
une paire de dispositifs de jonction (80) qui relient le bras (40) et les premiers accessoires (100), et
une structure dans laquelle chacun des premiers accessoires peut tourner par rapport à chacun des dispositifs de jonction autour d'un axe de rotation s'étendant dans la même direction ou pratiquement dans la même direction que la direction latérale du bras,
**caractérisé en ce que** :
le dispositif optique de croissance pileuse (10) comprend une structure dans laquelle chacun des dispositifs de jonction (80) peut tourner par rapport au bras (40) autour d'un axe de rotation s'étendant dans la même direction ou pratiquement dans la même direction que la direction longitudinale du bras (40).

2. Dispositif optique de croissance pileuse (10) selon la revendication 1, dans lequel
le second accessoire (200) est fixé à l'unité d'émission de lumière (30) ou au bras (40) de façon à ne pas perturber le trajet de la lumière émise en sortie de l'unité d'émission de lumière (30).

3. Dispositif optique de croissance pileuse (10) selon la revendication 1, dans lequel
une paire de bras (40) est incluse,
la paire de bras (40) comprend un bras gauche (40A) et un bras droit (40B),
l'un des premiers accessoires (100A) est fixé au bras gauche (40A),
l'autre premier accessoire (100B) est fixé au bras droit (40B),
une articulation gauche (70A). reliant le bras gauche (40A) et l'unité d'émission de lumière (30), et une articulation droite (70B) reliant le bras droit (40B) et l'unité d'émission de lumière (30) sont en outre incluses,
l'articulation gauche (70A) est configurée pour faire tourner le bras gauche (40A) par rapport à l'unité d'émission de lumière (30) de façon à modifier la distance existant entre le premier des premiers accessoires (100A) et l'autre premier accessoire (100B), et
l'articulation droite (70B) est configurée pour faire tourner le bras droit (40B) par rapport à l'unité d'émission de lumière (30) de façon à modifier la distance existant entre le premier des premiers accessoires (100A) et l'autre premier accessoire (100B).

4. Dispositif optique de croissance pileuse (10) selon la revendication 1, dans lequel
le second accessoire (200) est configuré pour générer une force de réaction de valeur prédéterminée ou supérieure lors de la déformation élastique du second accessoire (200).

5. Dispositif optique de croissance pileuse (10) selon la revendication 1, dans lequel
le second accessoire (200) est fixé à l'unité d'émission de lumière (30), et la force avec laquelle les premiers accessoires (100) maintiennent leur propre position est réglée pour être plus grande que la force nécessaire pour déplacer les premiers accessoires (100) sur la base de la force de réaction que procure le second accessoire (200).

6. Dispositif optique de croissance pileuse (10) selon la revendication 1, dans lequel
le bras (40) comprend en outre une structure télescopique configurée pour modifier la longueur du bras (40) lui-même, et un mécanisme de verrouillage (42) configuré pour conserver la longueur du bras (40), et
la force générée par le mécanisme de guidage (42) pour conserver la longueur du bras (40) est réglée pour être plus grande que la force qui étend le bras (40) sur la base de la force de réaction que procure le second accessoire (200).
